(19) Europäisches Patentamt
European Patent Office
Office européen des brevets

(11) **EP 2 305 122 A1**

(12) **EUROPEAN PATENT APPLICATION**

(43) Date of publication:
**06.04.2011 Bulletin 2011/14**

(51) Int Cl.:
**A61B 8/00** *(2006.01)* **A61B 5/00** *(2006.01)*

(21) Application number: **10180890.5**

(22) Date of filing: **28.09.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **30.09.2009 JP 2009227544**

(71) Applicant: **Fujifilm Corporation
Minato-ku
Tokyo (JP)**

(72) Inventor: **Osawa, Atsushi
Ashigarakami-gun
Kanagawa (JP)**

(74) Representative: **Klunker . Schmitt-Nilson . Hirsch
Patentanwälte
Destouchesstrasse 68
80796 München (DE)**

(54) **Ultrasonic diagnostic apparatus and ultrasonic diagnostic method**

(57) An ultrasonic probe (11) has a plurality of ultrasonic transducers (UTs) (27). Each UT transmits ultrasonic waves and receives echo waves. In a B/A coefficient acquisition mode, every time a transmission of the ultrasonic waves and reception of echo waves takes place, ultrasonic waves for heating are transmitted to heat an object of interest. An HI processor (55) calculates a non-linear B/A coefficient based on a harmonic component of a detection signal from the UT. The HI processor acquires information of changes in the B/A coefficient while the obj ect of interest is heated with the ultrasonic waves for heating. A DSC (53) displays the information of the B/A coefficient together with an ultrasonic image (60) on a monitor (15).

FIG. 3

EP 2 305 122 A1

**Description**

FIELD OF THE INVENTION

The present invention relates to an ultrasonic diagnostic apparatus and an ultrasonic diagnostic method for performing diagnosis of a lesion based on a B/A coefficient obtained from a harmonic component of echo waves.

BACKGROUND OF THE INVENTION

Medical diagnosis using an ultrasonic diagnostic apparatus is commonly performed. The ultrasonic diagnostic apparatus is composed of an ultrasonic probe and an ultrasonic observation device. At a tip of the ultrasonic probe, a plurality of ultrasonic transducers (hereinafter abbreviated as UTs) are arranged. Each UT is composed of a backing material, a piezoelectric element, a pair of electrodes, an acoustic matching layer, and an acoustic lens. The UTs transmit ultrasonic waves to a subject (human body) and receive echo waves therefrom. Thereby detection signals are outputted from the UTs. The detection signals are electrically processed in an ultrasonic observation device or imaging device. Thus, an ultrasonic image is produced.

Emission with scanning of ultrasonic waves produces an ultrasonic cross-sectional image. To produce the ultrasonic cross-sectional image, a mechanical scan method and an electronic scan method are known. In the mechanical scan method, the UTs are mechanically rotated, swung, or slid. In the electronic scan method, the UTs are arranged in an array (hereinafter referred to as UT array), and the UT to be driven is selectively switched or changed using an electronic switch, for example, a multiplexer.

The ultrasonic waves from the UTs become distorted as they propagate inside the body of a patient. As a result, the ultrasonic waves propagating through the inside of the body of a patient have a fundamental component having the original frequency and nth harmonic components having a frequency n times as high as that of the fundamental component. For example, when ultrasonic waves (5MHz) are transmitted from the UTs, the ultrasonic waves having a fundamental component (5 MHz) and second, third, fourth,... to nth harmonic components (10 MHz, 15 MHz, 20 MHz, ... to 5 x n MHz) propagate through the body of a patient. The UTs receive the echo waves, mostly composed of the fundamental component and partly the harmonic components.

Recently in the field of ultrasonic diagnosis, harmonic imaging attracts attention. The harmonic imaging uses the harmonic component of the echo waves for imaging. (see Japanese Patent Laid-Open Publication No. 08-0187245 corresponding to U.S. Patent No. 5,724,976, Japanese Patent Laid-Open Publication No. 11-155863, PCT Publication No. WO 00/30543 corresponding to Japanese Patent Application Publication No. 2002-530145 and U.S. Patent No. 6,645,145, Japanese Patent Laid-Open Publication No. 2003-169800, Japanese Patent Laid-Open Publication No. 2003-210464, PCT publication No. WO 2005/084267 corresponding to Japanese Patent Application Publication No. 2007-531357 and U.S. Patent No. 7,612,483). The harmonic imaging, known as THI (Tissue Harmonic Imaging) and CHI (Contrast Harmonic Imaging), is used for clinical examinations of various diseases. The THI creates images that are derived solely from the harmonic component of the echo waves. The CHI creates images that are derived from the harmonic components of the harmonic resonance and disruption of microbubbles of an ultrasonic contrast agent. with the analysis of the harmonic components, a B/A coefficient (also referred to as non-linear parameter or non-linear acoustic parameter B/A) is acquired. The B/A coefficient indicates properties specific to living tissue, for example, density and stiffness. Application of the B/A coefficient to a new method of diagnosing a lesion is expected.

Conventionally, elastography and ARFI(Acoustic Radiation Force Impulse) are well known as methods for observing stiffness of living tissue, which is specific to living tissue. However, since elastography is performed with an ultrasonic probe being pushed against the body of a patient, observation results vary among operators or doctors and patients. Accordingly, it is difficult to obtain quantitative and reproducible values. Specifically, ARFI requires to emit extremely strong sound waves called push pulse to the human body for observation. It has been pointed out that the push pulse has adverse effects of to the human body.

The B/A coefficient has been researched because it is capable of clearing up the problems of the above described elastography and ARFI. For example, "Reflection type ultrasonic nonlinear parameter imaging system for medical diagnoses" [Takuso SATO et al, Report of Research Results for Grant-in-Aid for Scientific Research of the Japanese Ministry of Education, Culture, Sports, Science and Technology (Research Project No: 61420032), 1986-1987] discloses a technique to apply sound waves called "pump waves" to living tissue to create images derived from distribution of magnitude (B/A coefficient) of perturbation of living tissue.

At present, the B/A coefficient is not effectively used for the diagnosis of a lesion because measurement of an absolute value of the B/A coefficient still has uncertain factors. An apparatus disclosed in "Reflection type ultrasonic nonlinear parameter imaging system for medical diagnoses" has not been developed for commercial use.

SUMMARY OF THE INVENTION

An object of the present invention is to provide an ultrasonic diagnostic apparatus and an ultrasonic diagnostic method in which a B/A coefficient is effectively utilized for diagnosis of a lesion.

According to the present invention, the inventors of the present invention focused on temperature dependence of the B/A coefficient to utilize this property for the diagnosis of a lesion. The temperature dependence of the B/A coefficient is disclosed in "In vitro measurement of B/A in animal tissue using thermodynamics" (Tetsuya ASAHINA, Nobuyuki ENDOH, Jpn. J. Med. Ultrasonics Vol.17 No.4 (1990) p.358). For example, the B/A coefficient of bovine fat increases 0.03 per degree rise in temperature. The B/A coefficient of bovine liver increases 0.05 per degree rise in temperature. The temperature dependence of the B/A coefficients of water, physiological saline solution, and agar is measured as reference samples. The B/A coefficients of water and physiological saline solution increase 0.028 per degree rise in temperature. The B/A coefficient of agar increases 0.032 per degree rise in temperature.

An ultrasonic diagnostic apparatus of the present invention includes an ultrasonic transducer, a harmonic image processor, and an acquisition section. The ultrasonic transducer transmits ultrasonic waves to an object of interest, and receive echo waves from the object of interest to output a detection signal. The harmonic image processor calculates a B/A coefficient based on a signal component corresponding to a harmonic component in the detection signal. The acquisition section acquires information on a change in the B/A coefficient relative to a temperature change of the object of interest.

It is preferable that the ultrasonic diagnostic apparatus further includes a display controller which makes a monitor to display the information acquired by the acquisition section.

It is preferable that the display controller makes the monitor to display an ultrasonic image produced based on a fundamental component of the detection signal.

It is preferable that the ultrasonic diagnostic apparatus further includes a temperature controller for changing the temperature of the object of interest.

It is preferable that the temperature controller heats the object of interest with sound waves.

It is preferable that the temperature controller heats the object of interest with ultrasonic waves.

It is preferable that the temperature controller is the ultrasonic transducer.

It is preferable that the ultrasonic diagnostic apparatus further includes a controller for controlling at least one of a level, a frequency, a transmission time, a transmission area, and a focal region of the ultrasonic waves to adjust an irradiation energy amount of the ultrasonic waves transmitted to the object of interest.

It is preferable that the ultrasonic diagnostic apparatus further includes a designation section for designating a region of interest from the object of interest, and the temperature controller selectively changes the temperature of the designated region of interest.

It is preferable that the acquisition section acquires at least one of the relative values of the B/A coefficient when the temperature of the object of interest is constant, a rate of increase of the B/A coefficient while the object of interest is being heated, and a rate of decrease of the B/A coefficient while the object of interest is being cooled from the acquired information.

An ultrasonic diagnostic method of the present invention includes a transmission step, a reception step, an extraction step, a calculation step, and information obtaining step. In the transmission step, ultrasonic waves are transmitted to an object of interest. In the reception step, the echo waves from the object of interest are received and a detection signal is outputted. In the extraction step, a signal component corresponding to a harmonic component is extracted from the detection signal. In the calculation step, a B/A coefficient is calculated based on the signal component. In the information obtaining step, the above steps are repeated while the temperature of the object of interest changes, and information on temporal changes in the B/A coefficient is obtained.

According to the present invention, temporal changes in the B/A coefficient are obtained while the temperature of the object of interest changes. The obtained B/A coefficient is effectively utilized for a diagnosis of a lesion.

BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:

Figure 1 is a perspective view of an ultrasonic diagnostic apparatus;
Figure 2 is a partly exploded perspective view of an ultrasonic transducer array;
Figure 3 is a block diagram of an electrical configuration of the ultrasonic diagnostic apparatus;
Figure 4 shows a monitor in a state of displaying a pop-up window;
Figure 5 shows the monitor in a state of displaying a pop-up window;

Figure 6 is a perspective view of an ultrasonic transducer array of another embodiment; and
Figures 7 is a block diagram of an electrical configuration of ultrasonic transducers of another embodiment.

DESCRIPTION OF THE PREFERRED EMBODIMENTS

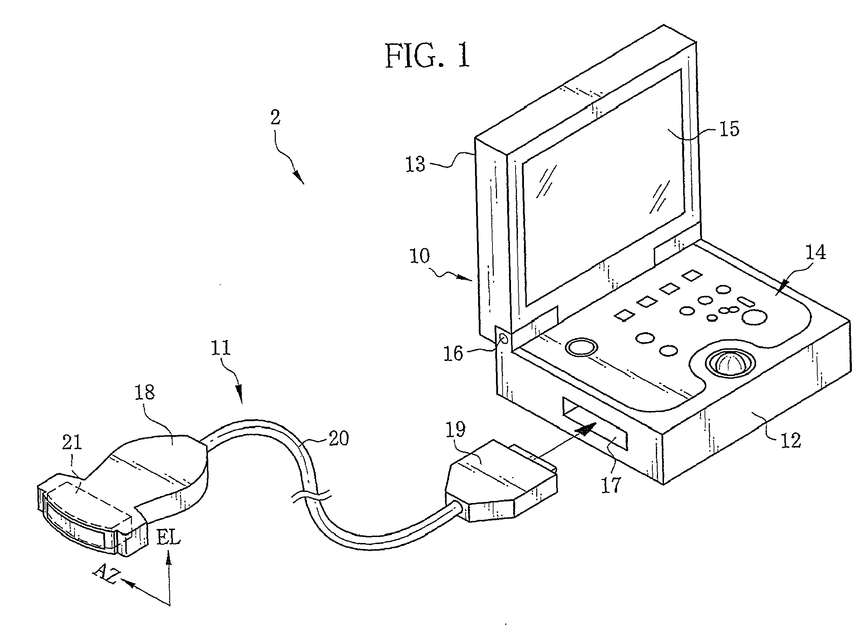

In Fig. 1, an ultrasonic diagnostic apparatus 2 is composed of a portable ultrasonic observation device 10 or imaging device and an external ultrasonic probe 11. The portable ultrasonic observation device 10 is composed of a housing 12 and a cover 13. An operation section 14 is provided on the top surface of the housing 12. The operation section 14 has buttons and a trackball to input various operation instructions to the portable ultrasonic observation device 10. On an inner surface of the cover 13, a monitor 15 is provided. The monitor 15 displays various operation screens including an ultrasonic image.

The cover 13 is attached to the housing 12 through a hinge 16. The cover 13 is rotatable between an open position and a closed position. In the open position, the operation section 14 and the monitor 15 are exposed. In the closed position, the inner surface and the top surface of the housing 12 face each other to cover the operation section 14 and the monitor 15 with each other. On a side of the housing 12, a grip (not shown) is provided. With the grip, the portable ultrasonic observation device 10 can be carried in the closed position. On the other side of the housing 12 opposite to the grip, a probe connector 17 is provided. The ultrasonic probe 11 is detachably connected to the probe connector 17.

The ultrasonic probe 11 is composed of a scan head 18, a connector 19, and a cable 20. The scan head 18 is held by an operator or doctor and gently pressed against a patient. The connector 19 is connected to the probe connector 17. The cable 20 connects the scan head 18 and the connector 19. At the tip of the scan head 18, an ultrasonic transducer array (hereinafter abbreviated as UT array) 21 is incorporated.

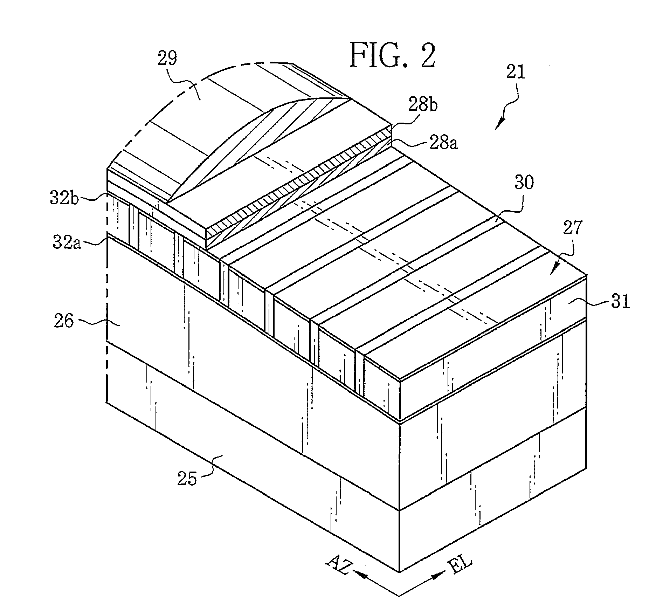

In Fig. 2, the UT array 21 has a flat base 25 made from glass-epoxy resin or the like, a backing material 26, ultrasonic transducers (hereinafter abbreviated as UTs) 27, acoustic matching layers 28a and 28b, and an acoustic lens 29 layered in this order from the bottom.

The backing material 26 is made from, for example, epoxy resin or silicone resin, and absorbs the ultrasonic waves emitted from the UTs 27 toward the base 25. The backing material 26 has a convex surface with a substantially dome-like cross-section in an elevation direction (hereinafter abbreviated as EL direction)(see Fig. 1).

Each of the UTs 27 has a plate-like shape, long in the EL direction. The UTs are spaced at regular intervals in an azimuth direction (scan direction of ultrasonic waves, hereinafter abbreviated as AZ direction) orthogonal to the EL direction. A filler 30 is filled between and around the UTs 27.

The acoustic matching layers 28a and 28b are made from, for example, epoxy resin. The acoustic matching layers 28a and 28b reduce a difference in acoustic impedance between the UTs 27 and the patient. The acoustic lens 29 is made from silicone resin or the like. The acoustic lens 29 converges the ultrasonic waves emitted from the UTs 27 onto an object of interest inside the body of the patient. It should be noted that the acoustic lens 29 may not be used. Instead of the acoustic lens 29, a protection layer may be provided.

Each UT 27 transmits ultrasonic waves and receives echo waves as a single channel. Since the UT array 21 is composed of the UTs 27 arranged in the AZ direction, the UT array 21 has multiple transmission and reception channels.

Each UT 27 has a piezoelectric ceramics thick film (inorganic piezoelectric element) 31 of PZT (lead zirconate titanate) sandwiched between first and second electrodes 32a and 32b. When a voltage (excitation pulse) is applied to the electrodes 32a and 32b, the inorganic piezoelectric element 31 oscillates or vibrates in the thickness direction to generate ultrasonic waves. Thereby, the ultrasonic waves are transmitted to an object of interest of the patient. When the UT 27 receives echo waves, the inorganic piezoelectric element 31 oscillates or vibrates to generate a voltage. The voltage is output as a detection signal from the UT 27 via the electrodes 32a and 32b.

The second electrode 32b is separated on a channel-by-channel basis, and individually provided for each UT 27. The first electrode 32a is provided all over the interface between the backing material 26 and the UTs 27. The first electrode 32a covers all the UTs 27.

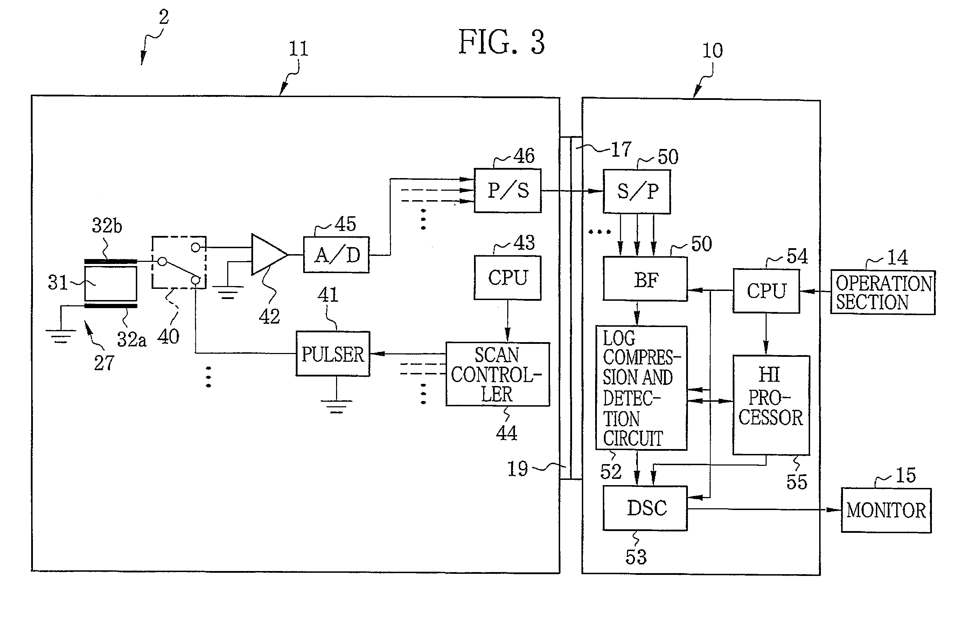

In Fig. 3, the first electrode 32a is connected to a ground. The second electrode 32b is connected to one end of a switch (hereinafter abbreviated as SW) 40. The SW 40 is a dual switch. A pulser 41 and a reception amplifier 42 are connected to the other ends of the SW 40.

Under the control of the CPU 43, the pulser 41 is driven by the scan controller 44. The scan controller 44 selects a group of pulsers 41 to be driven from among all the pulsers 41. The group of the pulsers 41 to be driven is changed at a predetermined time interval. To be more specific, for example, in the case where there are 128 transmission and reception channels, adjacent 48 channels are selected as a block to be driven. The channels are driven sequentially on a block-by-block basis. In each block, each of the UTs 27 is driven with a delay. Every time a transmission of ultrasonic waves and reception of echo waves takes place, the block to be driven is switched or changed. Because adjacent blocks partly overlap with each other, the blocks are switched such that the channels to be driven are shifted or changed at a pitch or interval of one to several channels. Based on the drive signal from the scan controller 44, the pulser 41 transmits the UT 27 an excitation pulse to generate the ultrasonic waves.

An A/D converter (hereinafter abbreviated as A/D) 45 is connected to an output end of the reception amplifier 42. The reception amplifier 42 may be a voltage feedback type or a charge storage type. The reception amplifier 42 amplifies the detection signal (detection voltage) outputted from the UT 27 that received echo waves. The A/D 45 converts the detection signal from the reception amplifier 42 into a digital signal. Although only a single group of one reception amplifier 42, one A/D 45, one pulser 41, and one SW 40 is shown in the drawing, this group is provided for each channel.

As shown in Fig. 3, to transmit the ultrasonic waves, the SW 40 is turned to the pulser 41 side, namely, the pulser 41 and the UT 27 are connected, while the UT 27 and the reception amplifier 42 are disconnected. When the excitation pulse is applied from the pulser 41 to the UT 27, ultrasonic waves are emitted from the surface of the acoustic lens 29.

To receive the echo waves, on the other hand, the SW 40 is turned to the reception amplifier 42 side to disconnect the pulser 41 and the UT 27, and connect the UT 27 and the reception amplifier 42. When the echo waves are incident on the surface of the acoustic lens 29, the detection signal corresponding to the echo waves is outputted from the UT 27. The detection signal outputted from the UT 27 mainly represents a fundamental component of the echo waves and includes a harmonic component. The switching operation of the SW 40 is controlled by the scan controller 44.

The A/D 45 is connected to a parallel/ serial converter (hereinafter abbreviated as P/S) 46. The P/S 46 converts the detection signal (parallel data) from each A/D 45 into serial data. The serial data is inputted to a serial/ parallel conversion circuit (hereinafter abbreviated as S/P) 50 of the portable ultrasonic observation device 10 through the cable 20, the connector 19, and the probe connector 17.

The S/P 50 converts the serial data sent from the ultrasonic probe 11 back into the original parallel data. A beamformer (hereinafter abbreviated as BF) 51 performs phase matching operation to the detection signal converted back into the parallel data. A log compression and detection circuit 52 performs log compression to the detection signal outputted from the BF 51 to detect its level (amplitude). The detection signal outputted from the log compression and detection circuit 52 is temporarily stored in a memory (not shown).

Under the control of a CPU 54, a digital scan converter (hereinafter abbreviated as DSC) 53 converts the detection signal into a TV signal. The TV signal is subjected to D/A conversion by a D/A converter (not shown), and thus an ultrasonic image is displayed on the monitor 15.

The CPU 54 controls overall operations of the portable ultrasonic observation device 10. The CPU 54 operates each section based on an operation input signal from the operation section 14. The CPU 54 controls power supply to the ultrasonic probe 11.

The ultrasonic diagnostic apparatus 2 is provided with a normal mode, a harmonic imaging mode (hereinafter abbreviated as HI mode), and B/A coefficient acquisition mode. In the normal mode, an ultrasonic image is generated solely from a fundamental component of the echo waves. In the HI mode, an ultrasonic image is generated using a harmonic component of the echo waves. In the B/A coefficient acquisition mode, a B/A coefficient (non-linear parameter B/A) of a region of interest (abbreviated as ROI) is acquired. The operation section 14 is operated to select the mode and to designate the ROI.

A harmonic imaging processor (hereinafter abbreviated as the HI processor) 55 is actuated in the HI mode and the B/A coefficient acquisition mode.

In the normal mode, the DSC 53 generates an ultrasonic image based on the detection signal, obtained by the UT 27, representing the fundamental component of the echo waves. On the other hand, in the HI mode, the HI processor 55 actuates. Through filtering, the HI processor 55 extracts a signal component representing or corresponding to the harmonic component of the echo waves, obtained by the UT 27, from the detection signal. The DSC 53 generates an ultrasonic image using the harmonic component based on the detection signal extracted by the HI processor 55. An ultrasonic image may be generated using a combination of the fundamental component and the harmonic component.

In the B/A coefficient acquisition mode, every time a transmission of ultrasonic waves and reception of echo waves takes place, the ultrasonic waves for heating are transmitted to heat the object of interest. The ultrasonic waves for heating are, for example, burst waves or continuous waves. The ultrasonic waves for heating differ from the ultrasonic waves for generating an ultrasonic image in various parameters such as a level, a frequency, and a transmission time. Needless to say, the above parameters are set such that an irradiation energy amount of the ultrasonic waves for heating remains within a range specified by a standard such as MI (Mechanical Index) or TI (Thermal Index) based on FDA 510k or IEC standards. The CPU 43 and the scan controller 44 drive the UTs 27 with predetermined parameters to transmit the ultrasonic waves for heating and to transmit the ultrasonic waves and receive the echo waves.

The irradiation energy amount of the ultrasonic waves may be adjusted by changing the number of the UTs 27 to be driven (radiation range) or by making a change in a focal region in the AZ direction or in the EL direction between the ultrasonic waves for heating and those for generating an ultrasonic image.

The object of interest is gradually heated with heat energy of the ultrasonic waves for heating every time a transmission of ultrasonic waves and reception of echo waves takes place. Within a range of allowable temperature limit of the living tissue, the object of interest is heated for a predetermined time with the ultrasonic waves for heating. When the heating stops, the temperature increase of the object of interest stops, and the object of interest starts to dissipate heat. After a while, the temperature of the object of interest returns to the original temperature.

In the B/A coefficient acquisition mode, the HI processor 55 monitors temporal changes of the B/A coefficient relative to temperature changes of the object of interest. The results are outputted to the DSC 53. First, the HI processor 55 calculates the B/A coefficient based on the signal component representing or corresponding to the harmonic component of the echo waves of the detection signal received by the UTs 27. For the calculation of the B/A coefficient, a mathematical expression (1) is used.

$$(1) \qquad P_2 = \left(1 + \frac{B}{2A}\right) \frac{P_0^2 \omega}{2\rho_0 C_0^3} \frac{e^{-2\alpha_1 Z} - e^{-\alpha_2 Z}}{\alpha_2 - 2\alpha_1}$$

In the mathematical expression (1), $P_2$ represents a level of generation of the second harmonic component (a level of the signal component representing the second harmonic component of the detection signal), $P_0$ represents a level of sound pressure of the ultrasonic waves, $\rho_0$ represents density of living tissue, $C_0$ represents propagation sound velocity of small amplitude ultrasonic waves inside the living tissue, $\alpha_1$ represents an attenuation coefficient of the fundamental component, $\alpha_2$ represents an attenuation coefficient of the second harmonic component. $P_2$ is derived from the detection signal acquired by the UTs 27. The rest of the parameters are known. Accordingly, B/A in the parentheses of the mathematical expression, that is, the B/A coefficient is calculated by substituting the parameters in the mathematical expression (1). The B/A coefficient indicates properties such as density and stiffness of living tissue.

The HI processor 55 obtains data of temporal changes in the B/A coefficient from immediately before the start of the transmission of the ultrasonic waves for heating until the object of interest returns to its original temperature after the transmission of the ultrasonic waves for heating stops. For example, the HI processor 55 creates table data of the B/A coefficient associated with the time at which the B/A coefficient is obtained. The HI processor 55 obtains the B/A coefficient (base value) immediately before the start of the transmission of the ultrasonic waves for heating, the rate of increase of the B/A coefficient during the transmission of the ultrasonic waves for heating, and the rate of decrease of the B/A coefficient until the object of interest returns to its original temperature after the transmission of the ultrasonic waves for heating stops. The HI processor 55 calculates data related to the B/A coefficient with respect to the ROI designated using the operation section 14, and the calculated data is outputted to the DSC 53.

The detection signals outputted from the log compression and detection circuit 52 are stored in the memory in a state that the detection signals are sorted according to the channel received and with the fundamental component and harmonic component separated from each other. The HI processor 55 reads the signal component of the detection signal representing the harmonic component corresponding to an ROI designated using the operation section 14 to obtain the B/A coefficient.

The B/A coefficient (base value) is, for example, a value standardized with a maximum or an average value of all the B/A coefficients of living tissue around the ROI. For example, in the case where the B/A coefficient of the ROI is "10", and the maximum or the average value of the B/A coefficients around the ROI is "12.5", the B/A coefficient (base value) of the ROI is 10/ 12.5 = 0.8. The harmonic component of the echo waves detected by the UTs 27 is at an extremely weak level, so the obtained B/A coefficient itself is not reliable. For this reason, the base value obtained by relative comparison of the B/A coefficients in different areas is used.

The rate of increase of the B/A coefficient is obtained by dividing the difference between the B/A coefficient obtained when the transmission of the ultrasonic waves for heating is stopped and the B/A coefficient immediately before the start of the transmission of the ultrasonic waves for heating, by the transmission time of the ultrasonic waves for heating. The rate of decrease of the B/A coefficient is obtained by dividing the difference between the B/A coefficient obtained when the transmission of the ultrasonic waves for heating is stopped and the B/A coefficient immediately before the start of the transmission of the ultrasonic waves for heating, by the time between when the transmission of the ultrasonic waves for heating stops and when the B/A coefficient returns to its original value. The rates of increase and decrease of the B/A coefficient describe tendency for heating and thermal diffusion, in other words, specific heat which depends on proximity of surrounding tissue and heat diffusion due to blood flow.

Generally, living tissue in a malignant tumor is stiffer than that in a benign tumor. Accordingly, the B/A coefficient (base value) of a malignant tumor becomes higher than that in a benign tumor. Compared to a benign tumor, a malignant tumor has a large amount of blood flow and is likely to diffuse heat. As a result, the rate of increase of the B/A coefficient in a malignant tumor is lower than that in the benign tumor, meaning that a malignant tumor is heated slower than the benign tumor. On the other hand, the rate of decrease of the B/A coefficient in a malignant tumor is higher than that in the benign tumor, meaning that a malignant tumor is cooled faster than the benign tumor. Temporal changes in the B/A coefficient, the base value of the B/A coefficient, the rates of increase and decrease of the B/A coefficient, and the like are used as indices for the diagnosis of a lesion.

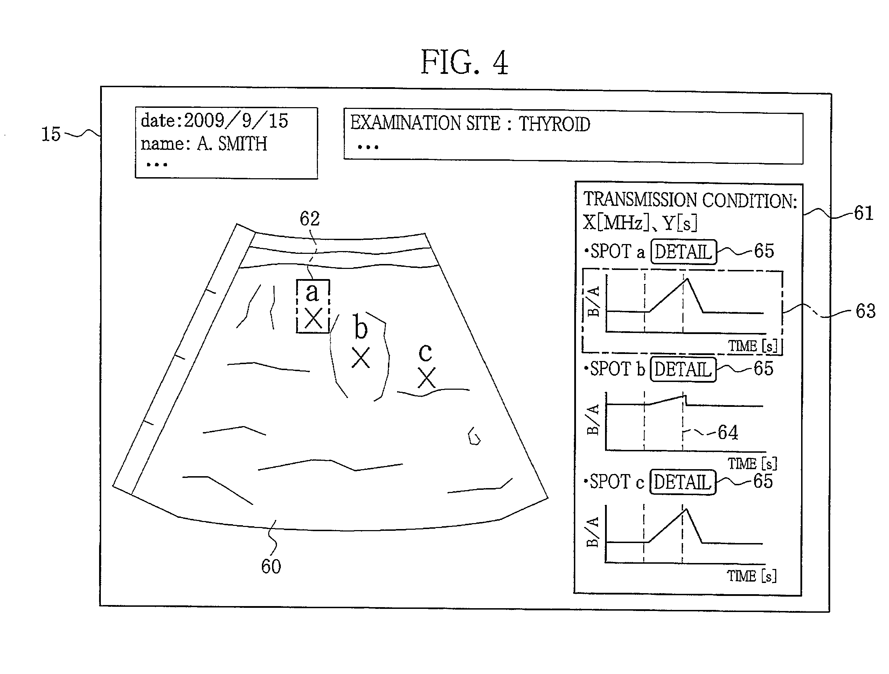

As shown in Fig. 4, in the B/A coefficient acquisition mode, a field 61 for displaying the B/A coefficient is displayed on a display screen on the monitor 15, in addition to an ultrasonic image 60 and information such as patient information and examination site. On the ultrasonic image 60, one or more markings 62 are superimposed. Each marking 62 is composed of a mark "x", indicating an ROI designated via the operation section 14, and a letter such as "a".

In the field 61 for displaying the B/A coefficient, graphs 63 of spots "a" to "c" in the ROI are displayed in addition to transmission status of the ultrasonic waves for heating. The transmission status includes, for example, frequency and transmission time. In the graph 63, the horizontal axis represents time and the vertical axis represents the B/A coefficient (base value). The graph 63 plots changes in the B/A coefficient (base value) with time based on the table data of time and B/A coefficient obtained from the HI processor 55. Inside the graph 63, dotted lines 64 show the transmission time.

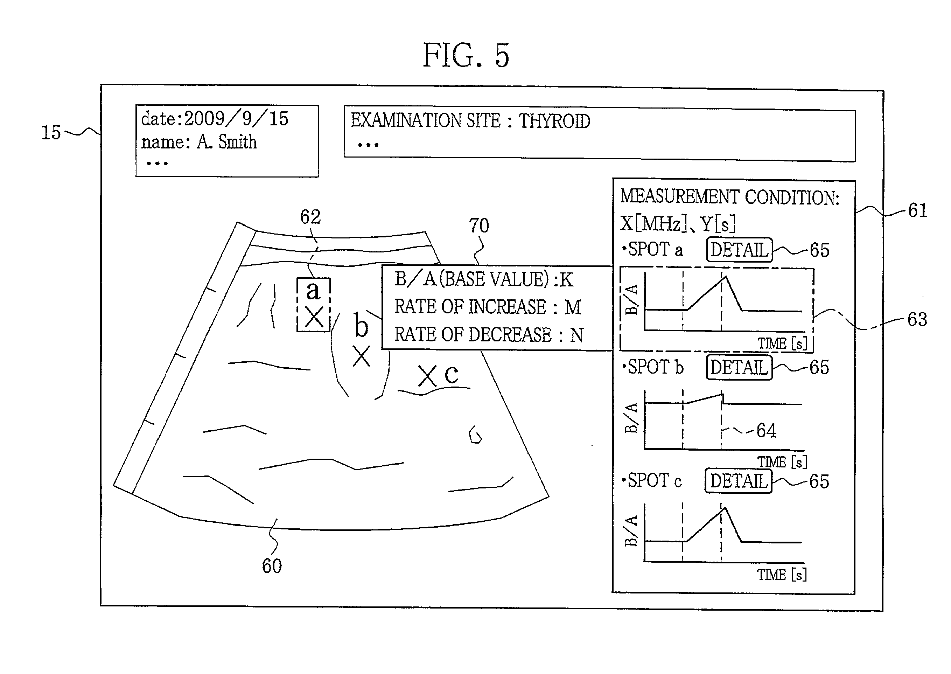

For the graph 63 of each spot, a detail button 65 is provided. When the detail button 65 is selected via the operation section 14, a pop-up window 70 shown in Fig. 5 appears on the side of the field 61 for displaying the B/A coefficient. In the pop-up window 70, the B/A coefficient (base value) and the rates of increase and decrease of the B/A coefficient are displayed in list form. In Fig. 5, a part of the ultrasonic image 60 is covered with the pop-up window 70 just for the sake of convenience in explanation. Actually, the pop-up window 70 is displayed in a layout not covering the ultrasonic image 60.

In graphs 63 of the spots "a" and "c", changes in the B/A coefficient are substantially the same. On the spot "b", changes in the B/A coefficient are obviously different from those on the spots "a" and "c". On the spot "b", the B/A coefficient (base value) is large. The rate of increase of the B/A coefficient is low, whereas the rate of decrease is high. A doctor analyzes changes in the B/A coefficient while observing the ultrasonic image 60, and uses the pop-up window 70 to check the B/A coefficient (base value) and the rates of increase and decrease of the B/A coefficient as necessary. Thus, diagnosis of the lesion is performed.

An operation of the ultrasonic diagnostic apparatus 2 having the above configuration is described. First, the connector 19 of the ultrasonic probe 11 is inserted and fixed in the probe connector 17 of the portable ultrasonic observation device 10. Thus, the portable ultrasonic observation device 10 and the ultrasonic probe 11 are connected. The operation section 14 is operated to turn on the portable ultrasonic observation device 10, and the power is supplied from the portable ultrasonic observation device 10 to the ultrasonic probe 11. The doctor observes an ultrasonic image displayed on the monitor 15 of the portable ultrasonic observation device 10 to perform diagnosis while he/she gently presses the scan head 18 of the ultrasonic probe 11 against the patient.

An excitation pulse is transmitted from the pulser 41 selected by the scan controller 44 of the ultrasonic probe 11 to the UT 27 of the corresponding channel. Thereby, the ultrasonic waves are emitted from the UT 27 to the patient. The scan controller 44 sequentially shifts the pulser 41 to be driven after a transmission of the ultrasonic waves and reception of the echo waves takes place. Thus, the patient is scanned with the ultrasonic waves. For the transmission, the scan controller 44 turns the SW 40, connected to the UT 27 which emits the ultrasonic waves, to the pulser 41 side.

The ultrasonic waves transmitted from the UTs 27 are reflected by the object of interest. The detection signal generated from the echo waves is outputted from the UT 27 of the corresponding channel. The scan controller 44 turns the SW 40, connected to the UT 27 for receiving the echo waves, to the reception amplifier 42 side. The detection signal from the UT 27 is amplified by the reception amplifier 42, and then subjected to the A/D conversion by the A/D 45. Thus, the detection signal is digitized. The digital detection signal is converted into serial data by the P/S 46, and then sent to the portable ultrasonic observation device 10.

In the portable ultrasonic observation device 10, the detection signal is converted back into the parallel data by the S/P 50. Then, the detection signal is sent to the BF 51 and subjected to the phase matching operation therein. Thereafter, the detection signal is subjected to the log compression and detection in the log compression and detection circuit 52, and then temporarily stored in the memory.

After the log compression and the detection, the detection signal is converted into the TV signal in the DSC 53. The TV signal is subjected to the D/A conversion and then displayed as an ultrasonic image on the monitor 15.

In the B/A coefficient acquisition mode, the ultrasonic waves for heating are emitted to the object of interest every time a transmission of the ultrasonic waves and reception of the echo waves takes place. Thus, the object of interest is heated. The HI processor 55 obtains the B/A coefficient from the signal component, representing the harmonic component of the echo waves, of the detection signal. Then, the HI processor 55 acquires the B/A coefficient (base value) and rates of increase and decrease of the B/A coefficient. The data is outputted to the DSC 53.

Based on the data from the HI processor 55, the DSC 53 controls the display of the B/A coefficient (base value) and the rates of increase and decrease of the B/A coefficient using a graph 63 in the field 61 for displaying the B/A coefficient and a pop-up window 70. The graph 63 and the pop-up window 70 are displayed on the monitor 15 together with the ultrasonic image 60.

As described above, attention is focused not on the B/A coefficient itself but on the temperature dependence of the B/A coefficient. While the object of interest is heated with the ultrasonic waves for heating, changes in the B/A coefficient are monitored. The results are displayed on the monitor 15. Thus, a new way of diagnosing a lesion using the B/A coefficient as an index becomes available.

Conventionally, elastography, ARFI, and color Doppler imaging are used in combination to examine stiffness of tissue and a state of blood flow. The present invention enables to examine the stiffness of tissue and a state of blood flow at a time.

The tissue may be judged normal or not, using a comparison between the acquisition result of the B/A coefficient and a predetermined threshold value. The judgment result may be displayed on the monitor or the like to notify the doctor.

In the above embodiment, the changes in the B/A coefficient are displayed using the field 61 for displaying the B/A coefficient and the pop-up window 70. Alternatively, the B/A coefficient (base value) and the rates of increase and decrease of the B/A coefficient may be superimposed on or around the marking 62 on the ultrasonic image 60. The ultrasonic image 60 may be displayed as a gray scale image to express the magnitudes of B/A coefficient (base value) and the rates of increase and decrease of the B/A coefficient with colors (for example, an area of the object of interest with maximum values may be colored in red, an area with medium values may be colored in pink, and an area with minimum values may be colored in white, and the like.). Temporal changes in the B/A coefficient (base value) may be expressed using lightness and darkness of a color, and the color may be superimposed on a moving image of the ultrasonic image 60 and made reproducible.

In the above embodiment, to heat the object of interest, the ultrasonic waves for heating are transmitted every time a transmission of the ultrasonic waves and reception of echo waves takes place. The number of cycles of transmission of the ultrasonic waves for heating can be changed. The ultrasonic waves for heating can be transmitted, for example, five times in every transmission and reception, or once in every 1 cycle of the transmission and reception. The B/A coefficient may be acquired several times with the different transmission conditions of ultrasonic waves for heating. The acquired data may be displayed in a comparative manner. Alternatively, the B/A coefficient may be obtained several times with the same transmission condition. An average of the acquired B/A coefficients may be obtained.

The ultrasonic waves for heating may be transmitted for a predetermined time. The transmission and reception of the ultrasonic waves may be performed only when the B/A coefficient starts to decrease. Thereby, only a rate of decrease is obtained. The ultrasonic waves for heating may be only transmitted to an area designated as an ROI.

In the above embodiment, the ultrasonic waves for heating are generated from the UTs for imaging. Alternatively, the UTs specifically used for transmitting the ultrasonic waves for heating may be provided. Instead of using ultrasonic waves, sound waves at a frequency of less than 20 kHz or electromagnetic waves (infrared rays) may be used, for example. Alternatively or in addition, a heater may be directly placed on the body surface.

Instead of heating the object of interest, the object of interest may be cooled. Changes in the B/A coefficient become the inverse of the above. Regardless of increase or decrease, changes in the B/A coefficient can be used as the index of the diagnosis of a lesion. To cool the object of interest, the ultrasonic probe may be provided with air supply/ water supply functions to apply cool air or cool water to the body. A cooling pad may be placed on the body surface.

In the above embodiment, the UTs with the inorganic piezoelectric ceramics thick film are used for transmission of the ultrasonic waves and the reception of the echo waves as an example. The UTs may have a different configuration.

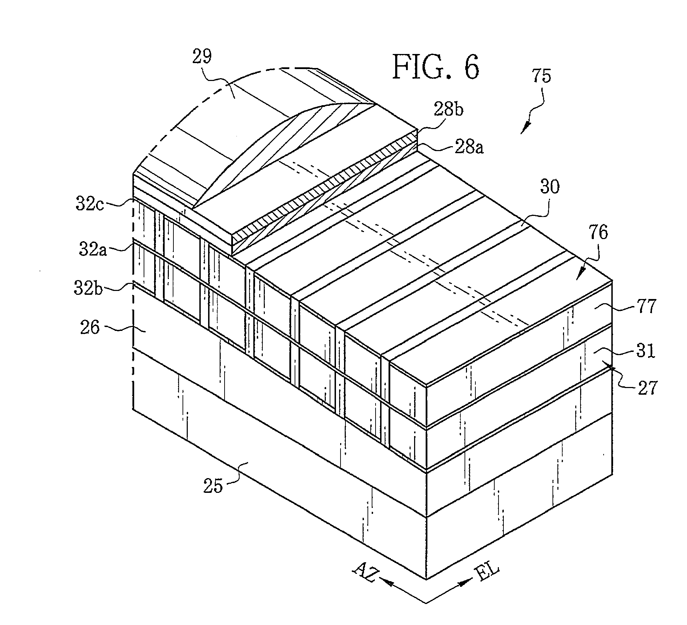

For example, a UT array 75 shown in Fig. 6 may be used. Basic configuration of the UT array 75 is the same or similar to that of the UT array 21 shown in Fig. 2. In the UT array 75, the UTs 27 are overlaid with the UTs 76. The UTs 27 are turned upside down from those shown in Fig. 2, namely, the first electrode 32a is on top of the UTs 27, and the second electrode 32b is on the bottom of the UTs 27.

Each UT 76 has an organic piezoelectric element 77 made from PVDF (Polyvinylidene difluoride) sandwiched between first and third electrodes 32a and 32c. The organic piezoelectric element 77 functions as the acoustic matching layer. Unlike the UTs 27, the UTs 76 only receive echo waves and do not transmit ultrasonic waves. The UTs 76 mainly output detection signals based on the harmonic component, for example, the secondary harmonic component of the echo waves.

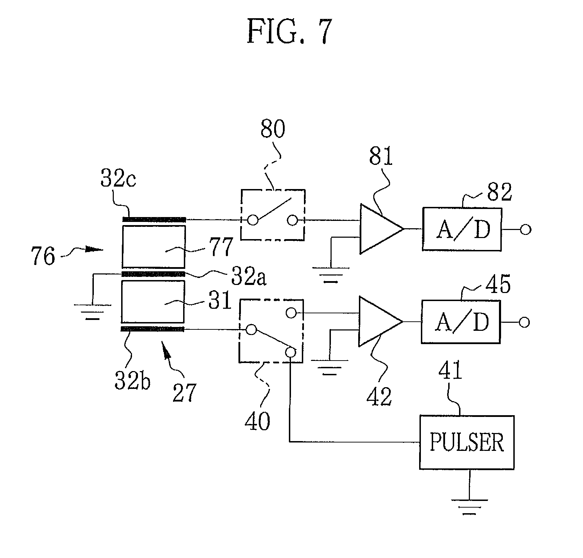

In Fig. 7, the third electrode 32c is connected to an end of a SW 80. The other end of the SW 80 is connected to a reception amplifier 81 and an A/D 82. The reception amplifier 81 is the same as or similar to the reception amplifier 42. The A/D 82 is the same as or similar to the A/D 45. To transmit the ultrasonic waves, the SW 80 is turned off. Conversely, to receive the echo waves, the SW 80 is turned on. The signal component, of the detection signal, representing or corresponding to the harmonic component of the echo waves received by the UTs 76 is inputted to the reception amplifier 81. The harmonic component is effectively acquired and acquisition accuracy of the B/A coefficient is improved when the UTs 76 using the organic piezoelectric element 77 are used for the reception of the harmonic component.

Alternatively, pMUT (Piezoelectric Micromachined Ultrasonic Transducer) may be used for the reception of the harmonic component. The pMUT has piezoelectric oxide thin film. Although the pMUTs are not suitable for the transmission of ultrasonic waves, they function sufficiently for the reception of echo waves. Additionally, other harmonic components including secondary harmonic components can be acquired by pMUT with changes in diameter and thickness. A dielectric constant of the pMUT is approximately 500 to 1000 times higher than that of organic piezoelectric elements such as PVDF. Because the pMUT has a membrane structure, capacitance of the pMUT is drastically higher than that of the organic piezoelectric elements. Accordingly, a level of the detection signal is higher than in the case where a material with relatively low capacitance is used. As a result, the harmonic component of the echo waves are effectively acquired.

In the above embodiment, the portable ultrasonic observation device and the ultrasonic probe are connected with the cable as an example. Alternatively, wireless data communication can be performed between the portable ultrasonic observation device and the ultrasonic probe. In this case, to transmit and receive the detection signals by radio, a wireless transmitter is provided at an output of the P/S 46, and a wireless receiver is provided at an input of the S/P 50. In addition, a battery is incorporated in the ultrasonic probe. The power from the battery is supplied to the each section of the ultrasonic probe.

A multiplexer may be inserted between the UT array and the pulser or between the pulser and the reception amplifier. The multiplexer selectively switches or changes the UTs to be driven. For example, in the case where 128 transmission and reception channels are used, and adjacent 48 channels are driven as one block, the multiplexer can select the block to be driven and adjust the delay timing of each UT. The number of pulsers is equal to the number of the channels to be driven at a time (in this case, 48). Thereby, the ultrasonic probe is further downsized. The scan control becomes easy because the scan controller only needs to transmit a switch signal to the multiplexer.

In the above embodiment, an external ultrasonic probe of a so-called convex electronic scan type is described as an example. Alternatively, an ultrasonic probe of a linear electronic scan type or a radial electronic scan type may be used. The present invention is also applicable to an internal ultrasonic probe inserted in a forceps channel of an electronic endoscope and an ultrasonic endoscope integrated with an electronic endoscope.

Various changes and modifications are possible in the present invention and may be understood to be within the present invention.

## Claims

1. An ultrasonic diagnostic apparatus (2) comprising:

   an ultrasonic transducer (27) for transmitting ultrasonic waves to an object of interest and for receiving echo waves from the object of interest to output a detection signal;
   a harmonic image processor (55) for calculating a B/A coefficient based on a signal component corresponding to a harmonic component in the detection signal;
   an acquisition section (55) for acquiring information on a change in the B/A coefficient relative to a temperature change of the object of interest.

2. The ultrasonic diagnostic apparatus of claim 1, further including a display controller (53) for making a monitor (15) to display the information acquired by the acquisition section.

3. The ultrasonic diagnostic apparatus of claim 2, wherein the display controller makes the monitor to display an ultrasonic image produced based on a fundamental component of the detection signal.

4. The ultrasonic diagnostic apparatus of claim 3, further including a temperature controller for changing temperature of the object of interest.

5. The ultrasonic diagnostic apparatus of claim 4, wherein the temperature controller heats the object of interest with sound waves.

6. The ultrasonic diagnostic apparatus of claim 4, wherein the temperature controller heats the object of interest with ultrasonic waves.

7. The ultrasonic diagnostic apparatus of claim 6, wherein the temperature controller is the ultrasonic transducer.

8. The ultrasonic diagnostic apparatus of claim 7, further including a controller (43, 44) for controlling at least one of a level, a frequency, a transmission time, a transmission area, and a focal region of the ultrasonic waves to adjust an irradiation energy amount of the ultrasonic waves transmitted to the object of interest.

9. The ultrasonic diagnostic apparatus of claim 4, further including a designation section (14) for designating a region of interest from the object of interest, and wherein the temperature controller selectively changes the temperature of the designated region of interest.

**10.** The ultrasonic diagnostic apparatus of claim 1, wherein the acquisition section acquires from the acquired information at least one of relative values of the B/A coefficient when the temperature of the object of interest is constant, a rate of increase of the B/A coefficient while the object of interest is being heated, and a rate of decrease of the B/A coefficient while the object of interest is being cooled.

**11.** An ultrasonic diagnostic method comprising the steps of:

     (A) transmitting ultrasonic waves to an object of interest;
     (B) receiving echo waves from the object of interest and outputting a detection signal;
     (C) extracting a signal component corresponding to a harmonic component from the detection signal;
     (D) calculating a B/A coefficient based on the signal component; and
     (E) repeating steps (A) to (D) while temperature of the object of interest changes and obtaining information on temporal changes in the B/A coefficient.

FIG. 1

EP 2 305 122 A1

FIG. 2

# FIG. 3

EP 2 305 122 A1

# FIG. 4

EP 2 305 122 A1

# FIG. 5

date:2009/9/15
name: A. Smith
...

EXAMINATION SITE : THYROID
...

MEASUREMENT CONDITION:
X[MHz]、Y[s]

·SPOT a    DETAIL    65

·SPOT b    DETAIL    65

·SPOT c    DETAIL    65

B/A (BASE VALUE) :K
RATE OF INCREASE : M
RATE OF DECREASE : N

FIG. 6

FIG. 7

EP 2 305 122 A1

**EUROPEAN SEARCH REPORT**

Application Number

EP 10 18 0890

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 936 308 A (FUKUKITA HIROSHI [JP] ET AL) 26 June 1990 (1990-06-26) | 1-6,10, 11 | INV. A61B8/00 A61B5/00 |
| Y | * column 1, line 28 - line 46 * <br> * column 4, lines 49-67 * <br> * column 8, line 47 - column 9, line 15 * <br> * column 15, lines 22-53 * <br> * figures 1, 2, 5 * | 7-9 | |
| X | EP 0 128 635 A2 (FUJITSU LTD [JP]) 19 December 1984 (1984-12-19) | 1-3,10, 11 | |
| A | * page 27; figure 21 * | 4-9 | |
| X | GONG X F ET AL: "Estimation Of Temperature Distribution In Biological Tissue By Acoustic Nonlinearity Parameter", INNOVATIONS IN NONLINEAR ACOUSTICS. INTERNATIONAL SYMPOSIUM ONNONLINEAR ACOUSTICS, AIP, US, vol. 838, 30 May 2006 (2006-05-30), pages 341-344, XP002471662, | 1-3,10, 11 | |
| A | * * pages 342-343 sections Principle of measurement, Experimentla method * <br> * figure 2 * * | 4-9 | TECHNICAL FIELDS SEARCHED (IPC) <br> A61B <br> A61N |
| X | EP 0 406 915 A1 (MATSUSHITA ELECTRIC IND CO LTD [JP]) 9 January 1991 (1991-01-09) | 1-6,10, 11 | |
| Y | * page 3, lines 8-12 * <br> * pages 12-13 * | 7-9 | |
| Y | US 2004/158152 A1 (LIZZI FREDERIC LOUIS [US] ET AL) 12 August 2004 (2004-08-12) | 7-9 | |
| A | * the whole document * | 1,4-6,11 | |
| Y | WO 2005/058138 A2 (ETHICON ENDO SURGERY INC [US]; MAST DOUGLAS T [US]; FAIDI WASEEM [US];) 30 June 2005 (2005-06-30) | 7-9 | |
| A | * paragraphs [0020] - [0022]; figures 2-4 * | 1,4-6,11 | |

The present search report has been drawn up for all claims

| Place of search | Date of completion of the search | Examiner |
|---|---|---|
| The Hague | 21 January 2011 | Dydenko, Igor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 18 0890

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2011

| Patent document cited in search report | | Publication date | Patent family member(s) | | Publication date |
|---|---|---|---|---|---|
| US 4936308 | A | 26-06-1990 | JP | 1299537 A | 04-12-1989 |
| | | | JP | 1725198 C | 19-01-1993 |
| | | | JP | 4028375 B | 14-05-1992 |
| EP 0128635 | A2 | 19-12-1984 | DE | 3475651 D1 | 19-01-1989 |
| | | | JP | 1731394 C | 29-01-1993 |
| | | | JP | 4020141 B | 31-03-1992 |
| | | | JP | 59164956 A | 18-09-1984 |
| | | | US | 4566460 A | 28-01-1986 |
| EP 0406915 | A1 | 09-01-1991 | DE | 3688702 D1 | 19-08-1993 |
| | | | DE | 3688702 T2 | 09-12-1993 |
| | | | EP | 0226466 A2 | 24-06-1987 |
| | | | US | 4817615 A | 04-04-1989 |
| US 2004158152 | A1 | 12-08-2004 | US | 2009287082 A1 | 19-11-2009 |
| WO 2005058138 | A2 | 30-06-2005 | EP | 1696799 A2 | 06-09-2006 |

EPO FORM P0459

For more details about this annex : see Official Journal of the European Patent Office, No. 12/82

**EP 2 305 122 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8187245 A
- US 5724976 A
- JP 11155863 A
- WO 0030543 A
- JP 2002530145 A
- US 6645145 B
- JP 2003169800 A
- JP 2003210464 A
- WO 2005084267 PCT
- JP 2007531357 A
- US 7612483 B

**Non-patent literature cited in the description**

- **Takuso SATO et al.** Report of Research Results for Grant-in-Aid for Scientific Research of the Japanese Ministry of Education, Culture, Sports, Science and Technology. Research Project No: 61420032, 1986
- **Tetsuya ASAHINA ; Nobuyuki ENDOH.** In vitro measurement of B/A in animal tissue using thermodynamics. *Jpn. J. Med. Ultrasonics,* 1990, vol. 17 (4), 358